# EUROPEAN PATENT APPLICATION

(11) **EP 1 142 868 A1**
(43) Date of publication of application: **10.10.2001**
(21) Application number: 99959909.5
(22) Date of filing: 20.12.1999
(51) Int. Cl.: C07C 271/28

(54) **AMIDE DERIVATIVES**

(30) Priority: 22.12.1998 JP 36449998
(71) Applicant: MITSUBISHI CHEMICAL CORPORATION, Chiyoda-ku, Tokyo 100-0005 (JP)
(72) Inventor: ANDO, Ryoichi, Yokohama-shi, Kanagawa 227-0033 (JP); CHIBA, Noriko, Yokohama-shi, Kanagawa 227-8502 (JP)
(74) Representative: Sternagel, Fleischer, Godemeyer & Partner Patentanwälte
(86) International application number: JP9907138
(87) International publication number: WO0037434

(57) **Abstract**

Amide derivatives represented by the following general formula (I) and their pharmaceutically acceptable salts, as well as solvates and hydrates thereof, which exhibit anti-*Helicobacter pylori* activity and are useful as active ingredients for pharmaceutical compositions. 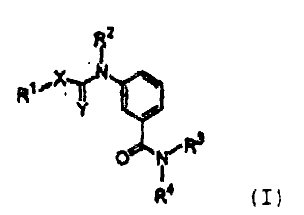 (wherein R¹ represents [1] a C₁-C₁₀ alkyl group optionally substituted with an optionally substituted C₆-C₁₄ aryl group, fluorenyl group or heterocycle residue, [2] an optionally substituted C₆-C₁₄ aryl group or [3] an optionally substituted heterocycle residue; R², R³ and R⁴ each independently represent a hydrogen atom or a C₁-C₅ alkyl group; X represents an oxygen atom or N-R⁵ (where R⁵ represents a hydrogen atom or a C₁-C₆ alkyl group); and Y represents an oxygen atom or sulfur atom. Excluded are compounds wherein R¹ is benzyl, R² and R³ are hydrogen atoms, R⁴ is propyl and X and Y are oxygen atoms.)

## Description

### Technical Field

The present invention relates to novel amide derivatives.

### Background Art

*Helicobacter pylori* is a slightly aerobic gram-negative bacterium which was recently isolated from human gastric mucosa, and various published reports suggest its involvement in inflammation of the alimentary tract, formation and recurrence of ulcer, and more over gastric cancer (Molecular Medicine, Vol. 31, pp. 1304-1374, 1994).

For the treatment of gastrointestinal ulcers, medicaments such as H₂ blockers or proton pump inhibitors have been used in the past, but since the aforementioned relation between *Helicobacter pylori* infection and gastric ulcers has gradually come to light, combinations of antibacterial agents such as clarithromycin and amoxicillin have become common, particularly from the standpoint of preventing recurrence. In most cases, however, ordinarily used antibacterial agents fail to achieve complete elimination of the bacteria, and in addition, they may affect the intestinal bacterial flora due to their broad antibacterial spectra, and often cause adverse effects such as diarrhea. It has therefore been desired to develop an antibacterial agent having potent antibacterial activity in the alimentary tract, which is specific against *Helicobacter pylori.*

The compound represented by general formula (I) defined herein below wherein R¹ is a benzyl group, R² and R³ are a hydrogen atom, R⁴ is a propyl group and X and Y are an oxygen atom, has been reported as synthetic intermediates in receptor models (Journal of American Chemical Society, Vol. 115, pp. 3548, 1993). However, it has not been known that the compound has antibacterial activity against *Helicobacter pylori*.

### Disclosure of the Invention

The inventors of the present invention conducted research to provide a novel anti-*Helicobacter pylori* agent, and as a result, have found that the compounds represented by the following general formula have excellent antibacterial activity against *Helicobacter pylori,* and can exhibit potent antibacterial activity in the alimentary tract. The present invention was achieved on the basis of these findings.

The present invention thus provides amide derivatives represented by the following general formula (I) and their pharmaceutically acceptable salts, as well as their solvates and hydrates. 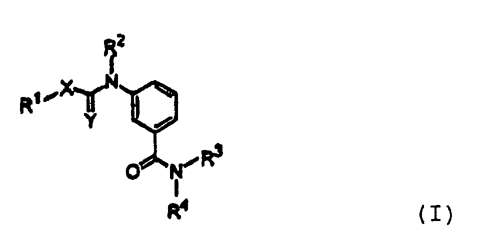 wherein R¹ represents [1] a C₁-C₁₀ alkyl group optionally substituted with an optionally substituted C₆-C₁₄ aryl group, fluorenyl group or optionally substituted heterocycle residue, [2] an optionally substituted C₆-C₁₄ aryl group or [3] an optionally substituted heterocycle residue; R², R³ and R⁴ each independently represent a hydrogen atom or a C₁-C₆ alkyl group; X represents an oxygen atom or N-R⁵ (where R⁵ represents a hydrogen atom or a C₁-C₅ alkyl group); and Y represents an oxygen atom or sulfur atom. However, this excludes compounds wherein R¹ is benzyl group, R² and R³ are a hydrogen atom, R⁴ is propyl group and X and Y are an oxygen atom.

According to another aspect of the present invention, there are provided medicaments, preferably for the prevention, treatment or avoidance of relapse of digestive diseases (e.g., gastritis, gastric ulcer, duodenal ulcer, gastric cancer, duodenal cancer, malignant lymphoma and MALT lymphoma), which comprise as active ingredients compounds selected from the group consisting of the aforementioned amide derivatives and their pharmaceutically acceptable salts, and solvates thereof and hydrates thereof. The medicaments are preferably provided as pharmaceutical compositions comprising the aforementioned compounds as active ingredients together with one or more pharmaceutically acceptable additives. These medicaments can also be used as *anti-Helicobacter pylori* agents for therapeutic and/or preventive treatment of digestive diseases related to *Helicobacter pylori* infection.

According to further aspect of the present invention, there are provided a method for treating digestive diseases related to *Helicobacter pylori* infection which comprises the step of administering to a mammal such as a human a therapeutically effective dose of a compound selected from the group consisting of the aforementioned amide derivatives and their pharmaceutically acceptable salts and solvates and hydrates thereof, and the use of a compound selected from the group consisting of the aforementioned amide derivatives, pharmaceutically acceptable salts thereof, and solvates and hydrates thereof in the manufacture of the above medicaments.

### Best Mode for Carrying Out the invention

In the general formula (I), as the C₁-C₁₀ alkyl groups defined by R¹ there may be mentioned methyl, ethyl, propyl, isopropyl, butyl, isobutyl, pentyl, isopentyl, hexyl, isohexyl, heptyl, octyl, nonyl and decyl group, and such alkyl group may be substituted with C₆-C₁₄ aryl group, fluorenyl group or heterocycle residue. As the C₆-C₁₄ aryl group substituting the alkyl group there may be mentioned C₆-C₁₄ aryl group such as phenyl, naphthyl and anthryl group. As the heterocycle residue substituting the alkyl group there may be mentioned residues of heterocyclic compounds containing 1 to 4 heteroatom(s) selected from among oxygen, sulfur and nitrogen atom and having 5 to 10 ring-membered atoms in total, such as a furan ring, dihydrofuran ring, tetrahydrofuran ring, pyran ring, dihydropyran ring, tetrahydropyran ring, benzofuran ring, dihydrobenzofuran ring, isobenzofuran ring, chromene ring, chroman ring, isochroman ring, thiophene ring, benzothiophene ring, pyrrole ring, pyrroline ring, pyrrolidine ring, imidazole ring, imidazoline ring, imidazolidine ring, pyrazole ring, pyrazoline ring, pyrazolidine ring, triazole ring, tetrazole ring, pyridine ring, pyridineoxide ring, piperidine ring, pyrazine ring, piperazine ring, pyrimidine ring, pyridazine ring, indolizine ring, indole ring, indoline ring, isoindole ring, isoindoline ring, indazole ring, benzimidazole ring, purine ring, quinolizine ring, quinoline ring, phthalazine ring, naphthylidine ring, quinoxaline ring, quinazoline ring, cinnoline ring, pteridine ring, oxazole ring, oxazolidine ring, isoxazole ring, isoxazolidine ring, thiazole ring, thiazylidine ring, isothiazole ring, isothiazolidine ring, dioxane ring, dithian ring, morpholine ring or thiomorpholine ring. As the C₆-C₁₄ aryl group and the heterocycle residue defined by R¹ there may be mentioned the same groups defined as substituents for the aforementioned C₁-C₁₀ alkyl groups.

The aryl group and heterocycle residue defined above may also have one or more substituents. As substituents for the aryl group andheterocycle residue there may be mentioned halogen atom such as fluorine, chlorine and bromine atom; C₁-C₅ alkyl group such as methyl, ethyl, propyl, isopropyl, butyl, isobutyl, sec-butyl, tert-butyl, pentyl, isopentyl, neopentyl and tert-pentyl group; C₇-C₁₅ aralkyl group such as benzyl, phenylethyl and naphthylmethyl group; trifluoromethyl group; C₁-C₅ alkoxy group such as methoxy, ethoxy, propoxy, isopropoxy, butoxy, isobutoxy, tert-butoxy, pentyloxy and isopentyloxy group; C₇-C₁₅ aralkyloxy group such as benzyloxy, phenylethyloxy and naphthylmethyloxy group; C₁-C₅ alkylenedioxy group such as methylenedioxy, ethylenedioxy and propylenedioxy group; hydroxyl group; nitro group; C₂-C₆ alkylcarbonyloxy group such as acetoxy, propionyloxy, butyryloxy and valeryloxy group; carboxyl group; C₂-C₆ alkoxycarbonyl group such as methoxycarbonyl, ethoxycarbonyl, propoxycarbonyl, isopropoxycarbonyl, butoxycarbonyl, isobutoxycarbonyl, tert-butoxycarbonyl and pentyloxycarbonyl group; C₇-C₁₅ aralkyloxycarbonyl group such as benzyloxycarbonyl, phenylethyloxycarbonyl and naphthylmethyloxycarbonyl group; oxo group; C₂-C₆ alkylcarbonyl group such as acetyl, propionyl, butyryl and valeryl group; amino group; C₁-C₅ monoalkylamino group such as methylamino, ethylamino, propylamino, isopropylamino, butylamino, isobutylamino, tert-butylamino, pentylamino and isopentylamino group; C₂-C₁₀ dialkylamino group such as dimethylamino, ethylmethylamino, diethylamino, methylpropylamino and diisopropylamino group; C₂-C₆ alkylcarbonylamino group such as acetylamino, propionylamino, isopropionylamino, butyrylamino and valerylamino group; C₂-C₆ alkoxycarbonylamino group such as methoxycarbonylamino, ethoxycarbonylamino, propoxycarbonylamino, isopropoxycarbonylamino, butoxycarbonylamino, isobutoxycarbonylamino, tert-butoxycarbonylamino and pentyloxycarbonylamino group; C₇-C₁₅ aralkyloxycarbonylamino group such as benzyloxycarbonylamino, phenylethyloxycarbonylamino and naphthylmethyloxycarbonylamino group; carbamoyl group; C₂-C₆ alkylcarbamoyl group such as methylcarbamoyl, ethylcarbamoyl, propylcarbamoyl, butylcarbamoyl, tert-butylcarbamoyl and pentylcarbamoyl group; and C₆-C₁₂ aryl groups such as phenyl and naphthyl.

R¹ is preferably a C₁-C₁₀ alkyl group optionally substituted with an optionally substituted C₆-C₁₄ aryl group or optionally substituted heterocycle residue. More preferably, it is a C₁-C₅ alkyl group optionally substituted with an optionally substituted C₆-C₁₄ aryl group or optionally substituted heterocycle residue, and even more preferably it is methyl group optionally substituted with an optionally substituted C₆-C₁₄ aryl group or optionally substituted heterocycle residue. As R¹ according to the present invention there is particularly preferred methyl group substituted with an optionally substituted C₆-C₁₄ aryl group or methyl group substituted with an optionally substituted heterocycle residue.

R², R³ and R⁴ each independently represent hydrogen atoms or C₁-C₅ alkyl group. As C₁-C₅ alkyl groups defined by R², R³ and R⁴ there may be mentioned such as methyl, ethyl, propyl, isopropyl, butyl, isobutyl, pentyl and isopentyl group. R² and R³ are preferably hydrogen atom*.* R⁴ is preferably an alkyl group, and even more preferably methyl group.

X represents an oxygen atom or N-R⁵ (where R⁵ represents a hydrogen atom or a C₁-C₅ alkyl group). As C₁-C₅ alkyl groups defined by R⁵ there may be mentioned such as methyl, ethyl, propyl, isopropyl, butyl, isobutyl, pentyl and isopentyl group. X is preferably an oxygen atom or N-H.

Y represents an oxygen atom or sulfur atom. Y is preferably an oxygen atom.

Preferred among the compounds of the present invention are those in which R² and R³ are hydrogen atom, X is an oxygen atom or N-H and Y is an oxygen atom, as well as their pharmaceutically acceptable salts or their solvates and hydrates. As more preferred compounds there may be mentioned those wherein R¹ is methyl group substituted with an optionally substituted C₆-C₁₄ aryl group or a heterocycle residue, R² and R³ are hydrogen atom, R⁴ is methyl group, X is an oxygen atom or N-H and Y is an oxygen atom, as well as their pharmaceutically acceptable salts and their solvates and hydrates.

As particularly preferred compounds there may be mentioned compounds selected from the group consisting:
N'-methyl-3-(2-chlorobenzyloxycarbonylamino) benzamide,
N'-methyl-3-(4-chlorobenzyloxycarbonylamino) benzamide,
N'-methyl-3-(2,3-dichlorobenzyloxycarbonylamino) benzamide,
N'-methyl-3-(2,6-dichlorobenzyloxycarbonylamino) benzamide,
N'-methyl-3-(2-bromobenzyloxycarbonylamino)benzamide
N'-methyl-3-(2-methylbenzyloxycarbonylamino) benzamide,
N'-methyl-3-(3-methylbenzyloxycarbonylamino) benzamide,
N'-methyl-3-(4-methylbenzyloxycarbonylamino) benzamide,
N'-methyl-3-(1-naphthylmethoxycarbonylamino) benzamide, and
N'-methyl-3-(2-naphthylmethoxycarbonylamino) benzamide,
and their pharmaceutically acceptable salts, or solvates and hydrates thereof.

The amide derivatives of the present invention represented by general formula (I) above can form pharmaceutically acceptable salts. Where one or more acidic groups exist, specific examples of salts will include metal salts such as lithium salts, sodium salts, potassium salts, magnesium salts and calcium salts, and ammonium salts such as inorganic ammonium salts, methylammonium salts, dimethylammonium salts, trimethylammonium salts and dicyclohexylammonium salts; where one or more basic groups exist, specific examples of salts will include mineral acid salts such as hydrochlorides, hydrobromides, sulfates, nitrates, and phosphates, and organic acid salts such as methanesulfonates, benzenesulfonates, para-toluenesulfonates, acetates, propionates, tartrates, fumarates, maleates, malates, oxalates, succinates, citrates, benzoates, mandelates, cinnamates and lactates. The amide derivatives of the present invention represented by general formula (I) may also exist as solvates or hydrates.

As for the stereochemistry of asymmetric carbon atoms present in the amide derivatives of the present invention represented by general formula (I), the atoms can independently be in the (R), (S), or (RS) configuration.

Specific examples of the amide derivatives of the present invention represented by general formula (I) include those listed in the following Table 1.

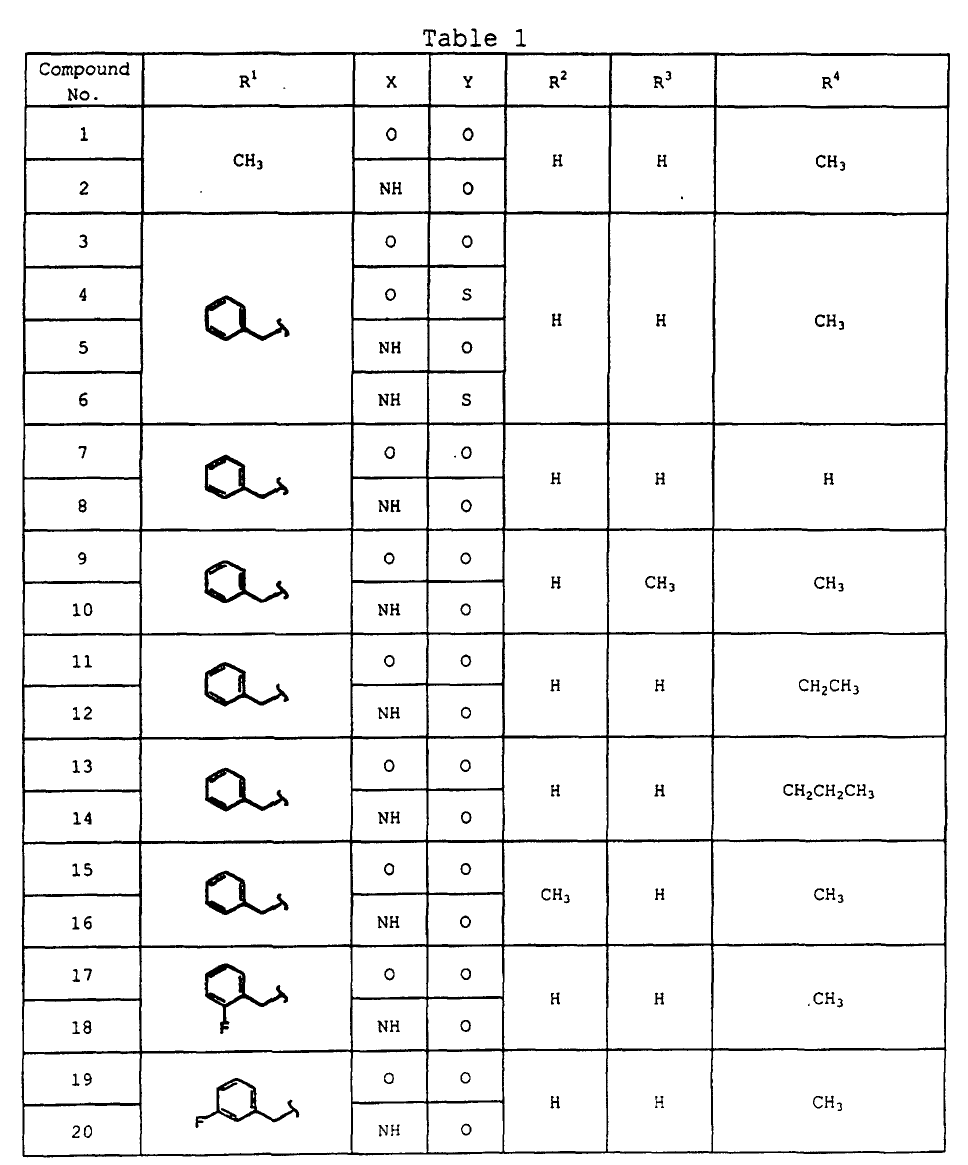

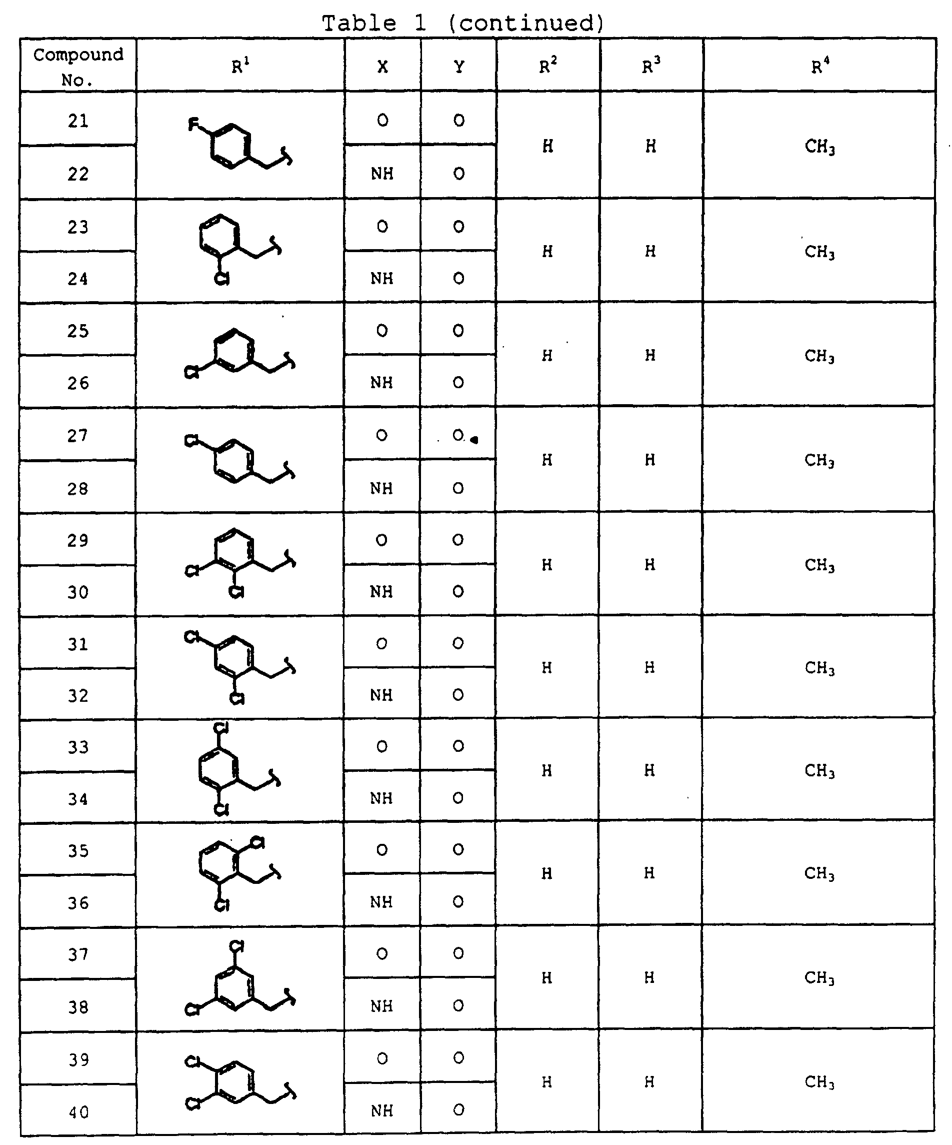

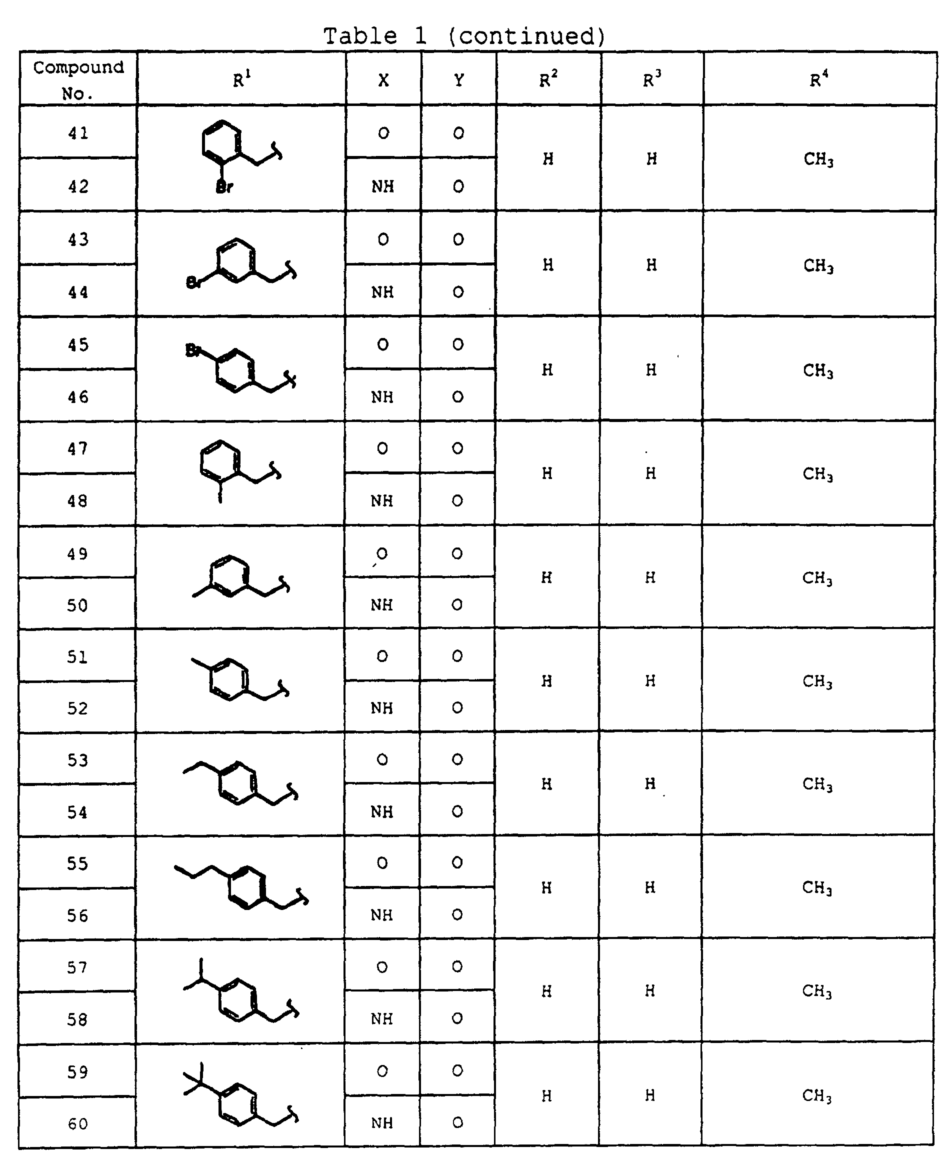

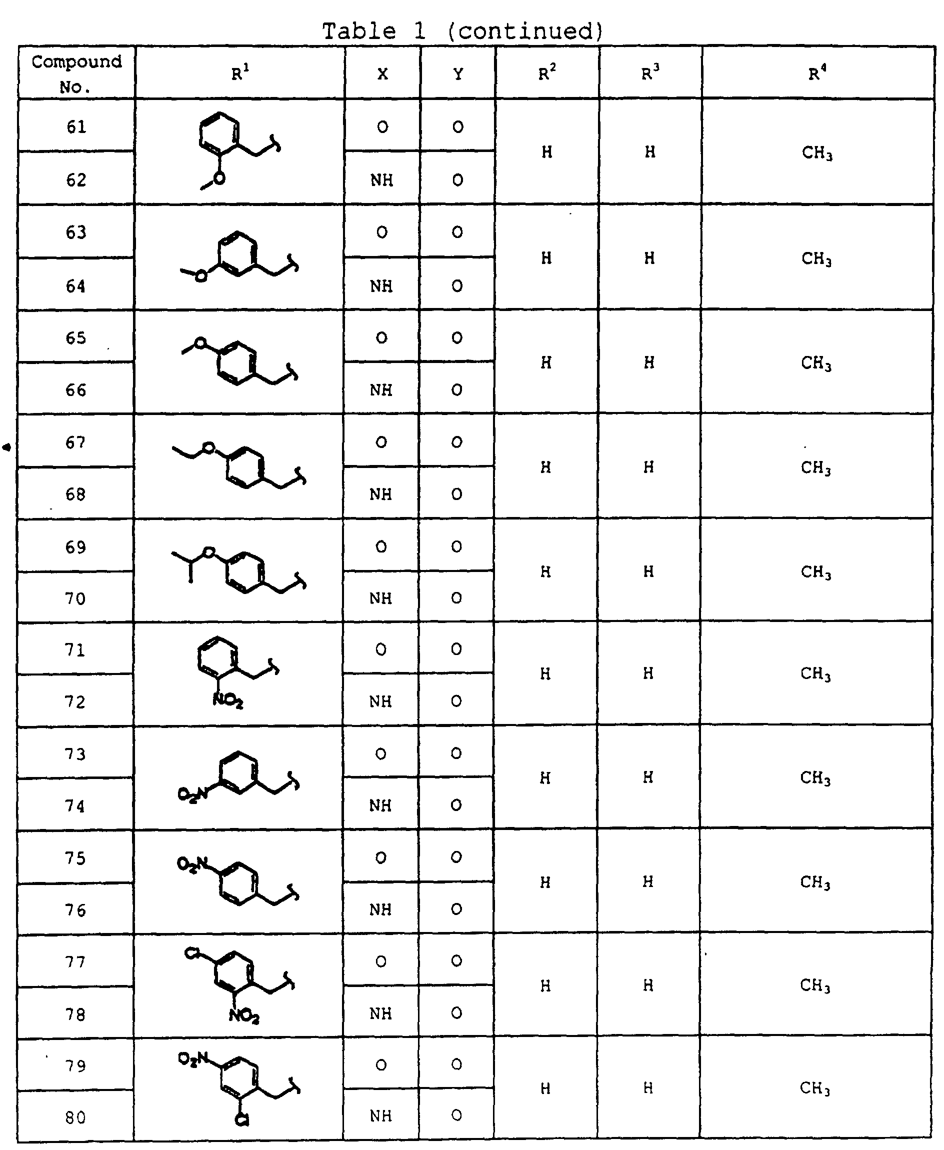

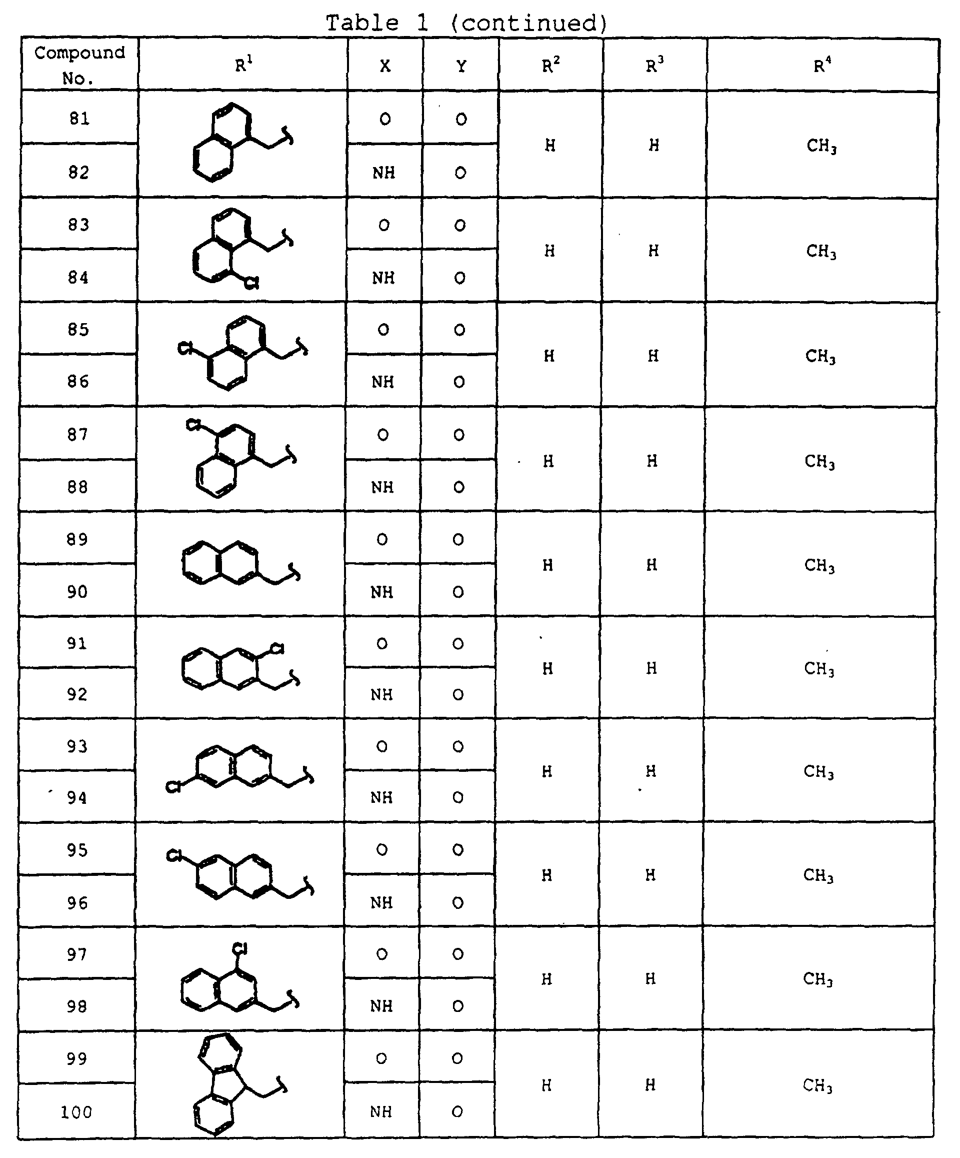

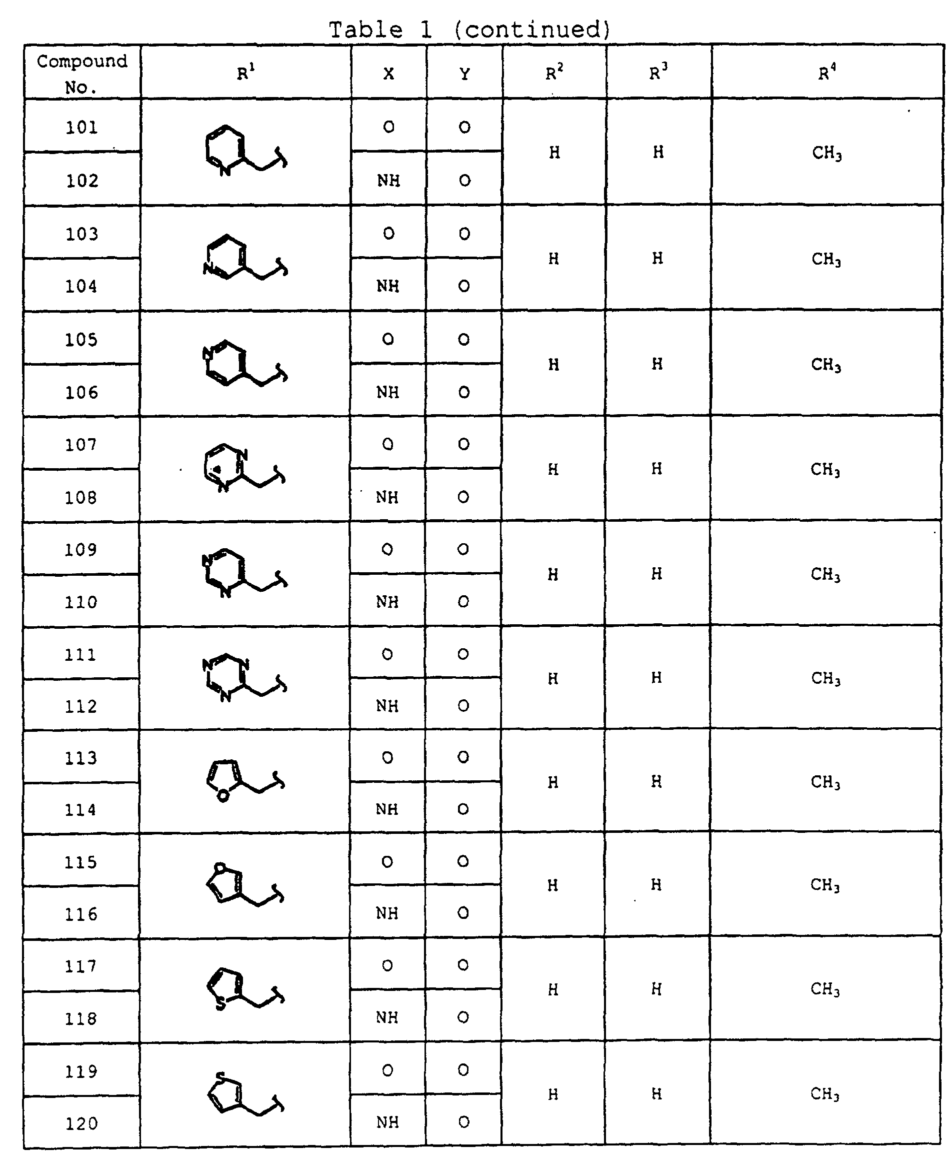

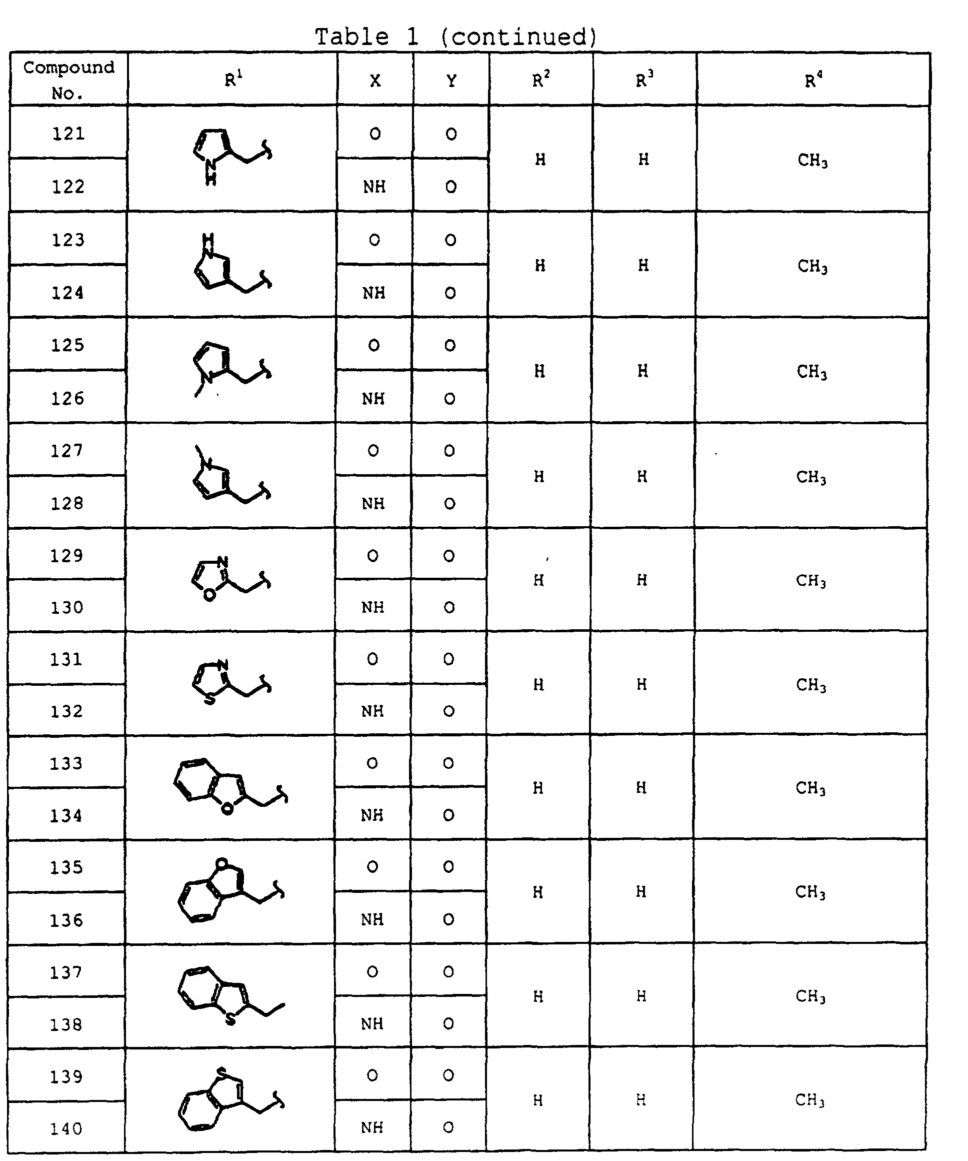

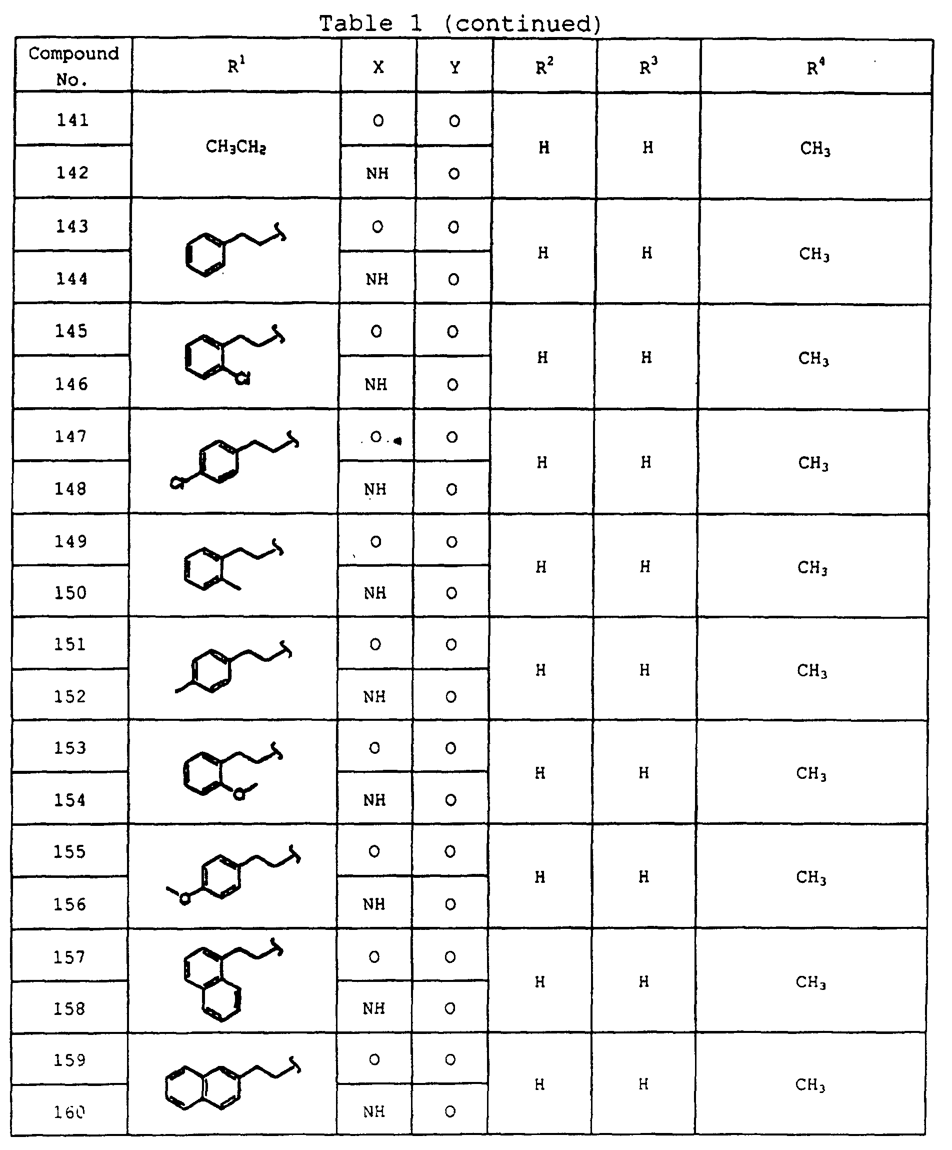

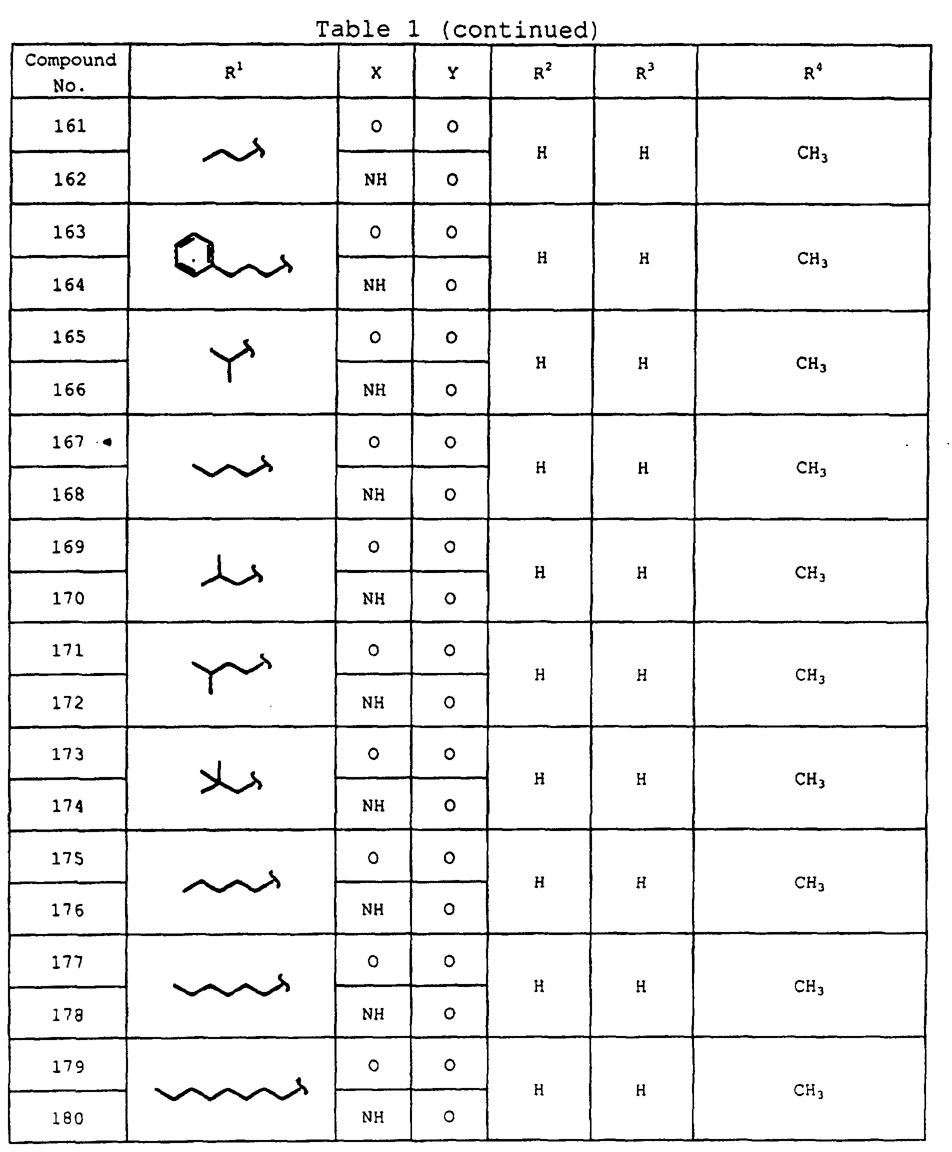

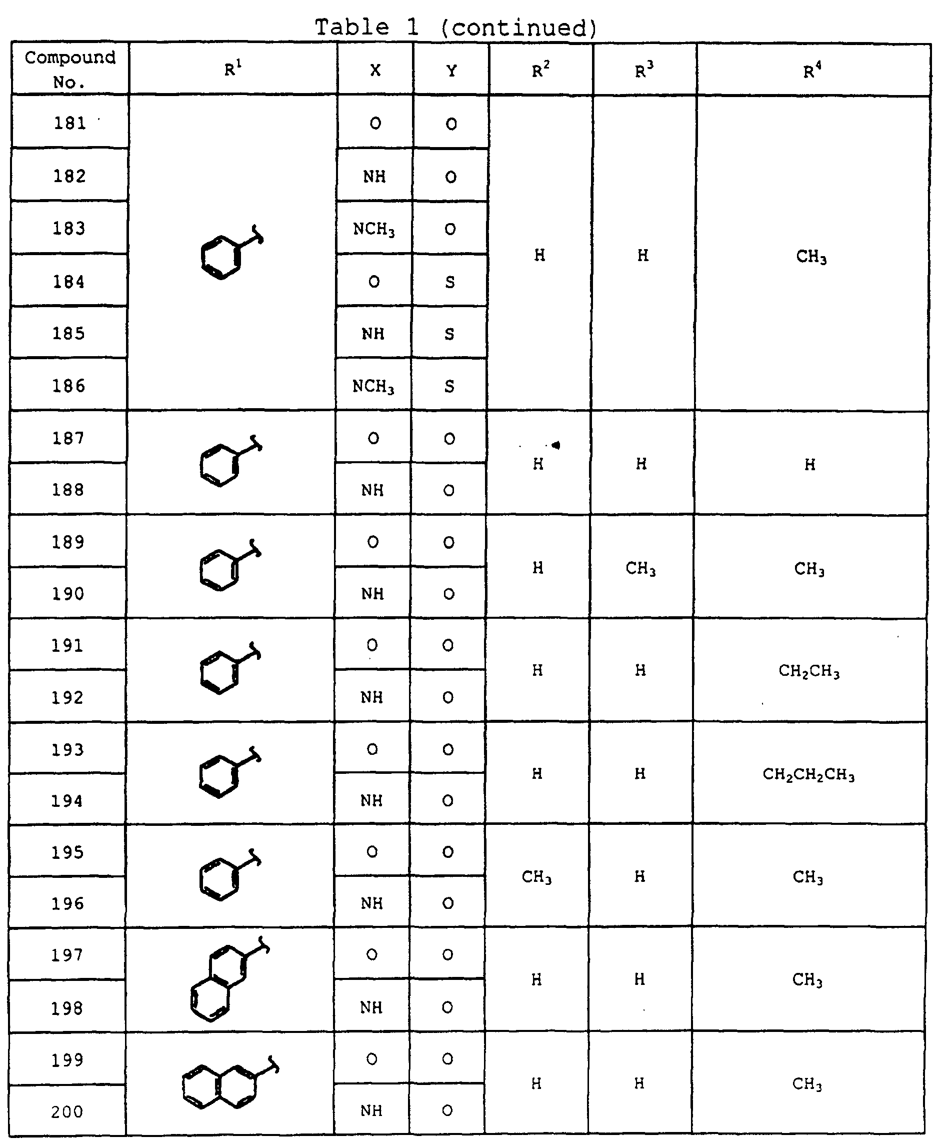

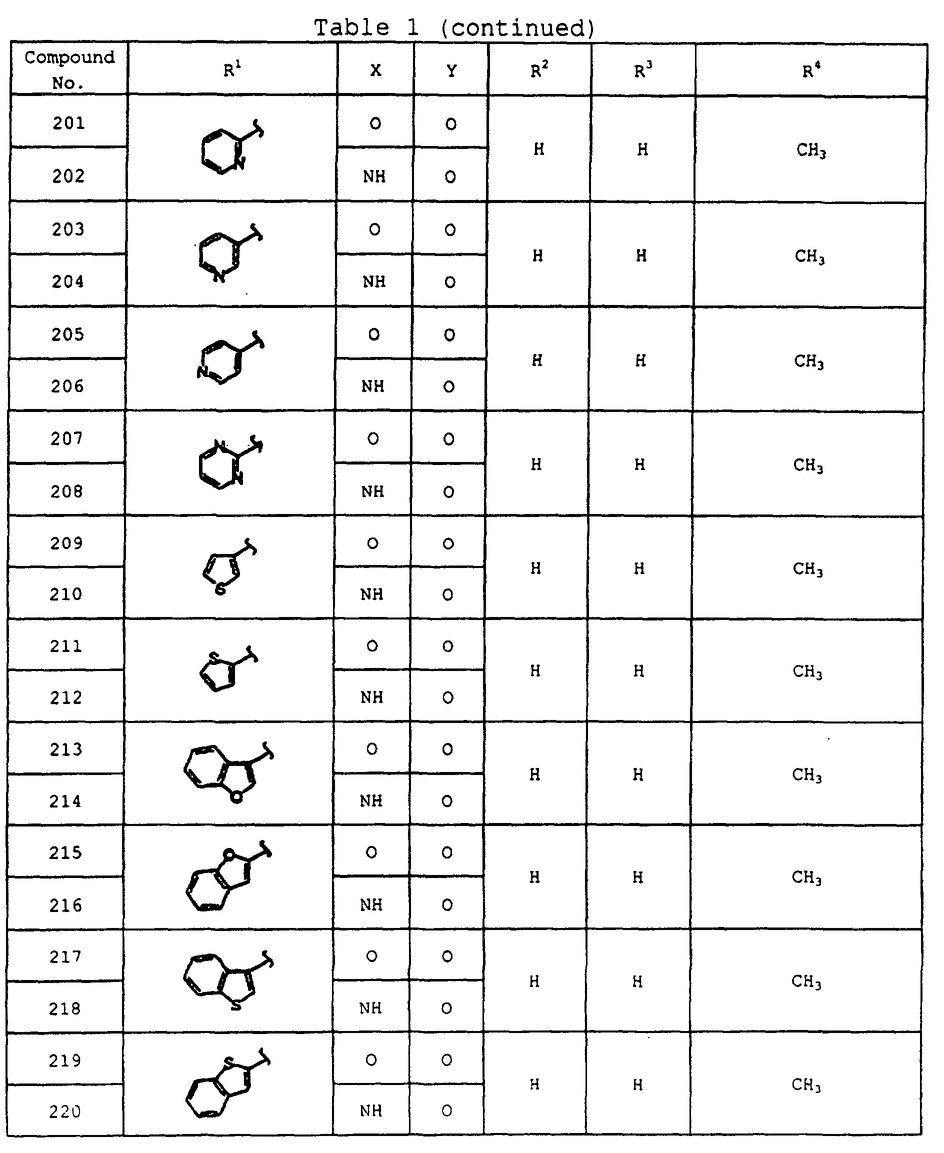

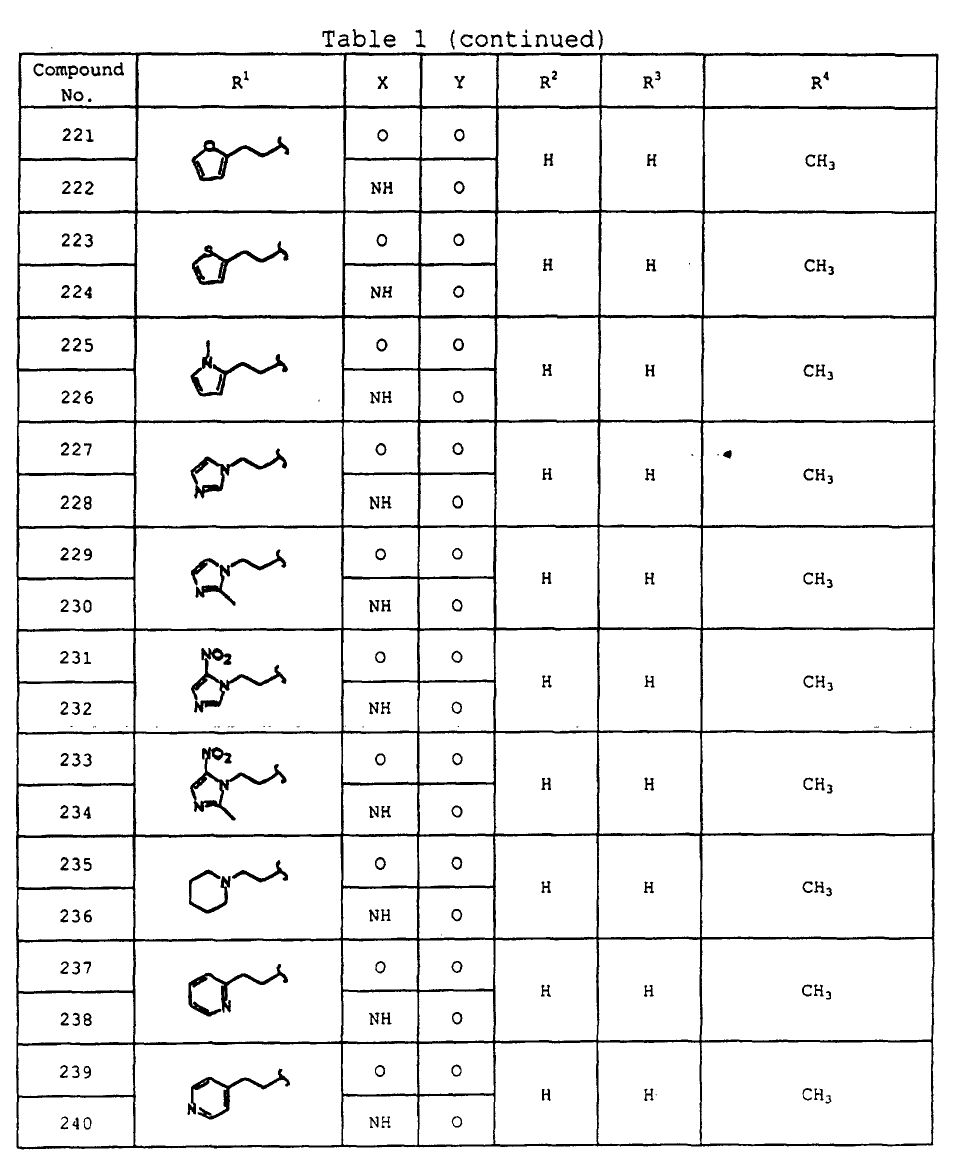

A production process for the compounds of the present invention will now be explained. The compounds represented by general formula (I) above may be produced, for example, by the following process.

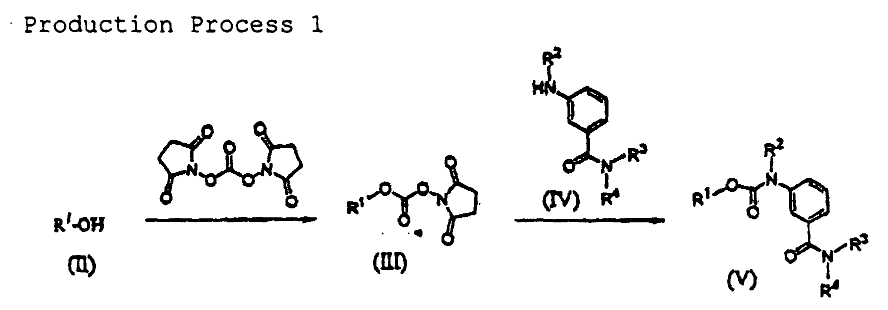

(R¹, R², R³ and R⁴ in the above general formulas have already been defined.)

An alcohol derivative represented by general formula (II) above is dissolved in an inert solvent such as acetonitrile, methylene chloride or chloroform, and allowed to be reacted with di(N-succinimidyl) carbonate in the presence of a base such as triethylamine or pyridine to obtain an asymmetric carbonate compound (III) as an intermediate. The compound (III) is then dissolved in a polar solvent such as dimethylformamide, N-methylpyrrolidone or dimethylsulfoxide, and allowed to react with the aniline derivative represented by general formula (IV) above in the presence of a base such as triethylamine or pyridine to obtain a compound represented by general formula (V) (the compound represented by formula (I) wherein X and Y are both oxygen atoms).

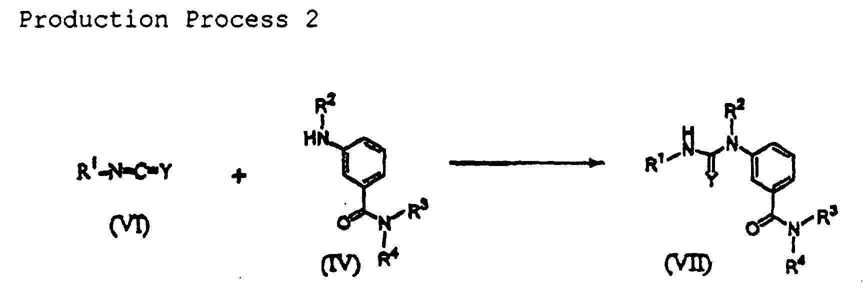

(R¹, R², R³, R⁴ and Y in the above general formulas have already been defined.)

An isocyanate derivative represented by general formula (VI) above is dissolved in an inert solvent such as acetonitrile, methylene chloride or chloroform and allowed to be reacted with an aniline derivative represented by general formula (IV) to obtain a compound represented by general formula (VII) (the compound represented by formula (I) wherein X is an NH group) .

In the above series of reactions, protection and deprotection of the functional groups may sometimes be required, and in such cases, a protective group suitable for each of the reactive functional groups may be chosen, and reaction procedures can be employed according to known methods described in the literature.

When the compound of the present invention is used clinically, its proportion with respect to the carrier component for the active ingredient for therapy will vary between 1 wt% and 90 wt%. The compound of the present invention may be administered, for example, as a pharmaceutical composition for oral administration in such forms as granules, subtilized granules, powders, hard capsules, soft capsules, syrups, emulsions, suspensions and liquid drugs, or administered as an injection for intravenous, intramuscular or subcutaneous administration. It may also be used in the form of a suppository. It may also be prepared as a powdery pharmaceutical composition for injection to be dissolved before use. Organic or inorganic pharmaceutical solid or liquid carriers or diluents suitable for oral, enteral or parenteral use may also be used to prepare the pharmaceutical compositions of the present invention. Examples of excipients used for manufacturing solid preparations include lactose, sucrose, starch, talc, cellulose, dextrin, kaolin and calcium carbonate. For the manufacture of liquid formulations for oral administration such as emulsions, syrups, suspensions, and liquids, ordinary inert diluents such as water and vegetable oils may be used. In addition to the inert diluents, auxiliaries such as, for example, moistening agents, suspending aids, sweetening agents, aromatics, colorants, preservatives and the like may be included in the formulations. Liquid formulations may be filled into capsules made of an absorbable material such as gelatin. Examples of solvents or suspending mediums used for the manufacture of pharmaceutical preparations for parenteral administration such as injections and suppositories include, for example, water, propylene glycol, polyethylene glycol, benzyl alcohol, ethyl oleate, lecithin and the like. Examples of base materials used for suppositories include, for example, cacao butter, emulsified cacao butter, lauric lipid, Witepsol and the like. Any common methods may be used for formulation of these preparations.

The clinical dose will generally be from about 0.01 to 5,000 mg per day for an adult based on weight, for oral administration, but the dose is preferably increased or decreased as appropriate depending on the age, conditions, and symptoms of the patient. The daily dose of the pharmaceutical composition of the present invention may be administered once a day or two to three times a day with suitable intervals, or alternatively, it may be administered intermittently.

When used as an injection, the dose of the compound of the present invention may be about 0.001 to 100 mg per day for an adult based on weight, either continuously or intermittently.

### Examples

The present invention will now be explained in more detail by way of the following examples, with the understanding that it is in no way limited by these examples insofar as the gist of the present invention is maintained.

### Example 1: Preparation of N'-methyl-3-(4-methylbenzyloxy carbonylamino)benzamide (Compound No.51 in Table 1)

After dissolving 307 mg of 4-methylbenzylalcohol and 966 mg of di(N-succinimidyl) carbonate in 20 ml of methylene chloride, adding 0.70 ml of triethylamine and stirring for 4 hours at room temperature, water was added. The aqueous layer was extracted with methylene chloride, and the extract was successively washed with saturated saline, saturated sodium bicarbonate, saturated saline, 2N hydrochloric acid and saturated saline and then dried with magnesium sulfate. After removing the magnesium sulfate by filtration, the filtrate was concentrated to obtain 664 mg of N-(4-methylbenzyloxycarbonyloxy)succinate imide as an intermediate.

A 610 mg portion of this product was dissolved in 2 ml of dimethylformamide, and 313 mg of 3-aminobenzoylmethylamide and 0.32 ml of triethylamine were added thereto, and then after stirring overnight at room temperature, the mixture was added to 15 ml of water while filtering out the insoluble product. The crystals produced from the filtrate were filtered and washed with water to obtain crude crystals. The crude crystals were dried and added to 8 ml of ethyl acetate, and then heated under reflux for 10 minutes. The mixture was cooled to room temperature and then filtered and washed with ethyl acetate to obtain 167 mg of the desired compound (27% yield).

Melting point: 167-168°C
IR (KBr, cm⁻¹) : 3322, 1738, 1622, 1557.
NMR (DMSO - d₆, δ) : 2.28 (s, 3H), 2.74 (d, J = 4.6Hz, 3H), 5.09 (s, 2H), 7.17 (d, J = 7.9Hz, 2H), 7.23 - 7.42 (m, 4H), 7.54 (d, J = 6.5Hz, 1H), 7.91 (s, 1H), 8.31 (d, J= 4.6Hz, 1H), 9.82 (s, 1H)

Compounds for Examples 2 to 18 and Example 22 were synthesized by the same process as Example 1. Their physical properties are listed below.

### Example 2: Preparation of N'-methyl-3-(2-fluorobenzyloxy carbonylamino)benzamide (Compound No.17 in Table 1)

Melting point: 189-190°C
IR (KBr, cm⁻¹) : 3341, 3291, 1730, 1622, 1557.
NMR (DMSO - d₆, δ) : 2.76 (d, J = 4.2Hz, 3H), 5.22 (s, 2H), 7.20 - 7.45 (m, 5H), 7.50 - 7.60 (m, 2H), 7.94 (s, 1H), 8.36 (d, J = 4.2Hz, 1H), 9.93 (s, 1H)

### Example 3: Preparation of N'-methyl-3-(4-fluorobenzyloxy carbonylamino)benzamide (Compound No.21 in Table 1)

Melting point: 153°C
IR (KBr, cm⁻¹) : 3304, 1732, 1626, 1613, 1559.
NMR (DMSO - d₆, δ) : 2.76 (d, J = 3.4Hz, 3H), 5.15 (s, 2H), 7.23 (dd, J = 8.6Hz, 8.6Hz, 2H), 7.35 (dd, J = 7.7Hz, 7.4Hz, 1H), 7.42 (d, J = 7.4Hz, 1H), 7.49 (dd, J = 8.6Hz, 8.6Hz, 2H), 7.57 (d, J = 7.7Hz, 1H), 7.94 (s, 1H), 8.36 (d, J = 3.4Hz, 1H), 9.90 (s, 1H)

### Example 4: Preparation of N'-methyl-3-(2-chlorobenzyloxy carbonylamino)benzamide (Compound No.23 in Table 1)

Melting point: 168°C
IR (KBr, cm⁻¹) : 3329, 3289, 1728, 1622, 1559.
NMR (DMSO - d₆, δ) : 2.74 (d, J = 4.4Hz, 3H), 5.23 (s, 2H), 7.27 - 7.43 (m, 4H), 7.43 - 7.60 (m, 3H), 7.93 (s, 1H), 8.30 (d, J = 4.4Hz, 1H), 9.94 (s, 1H)

### Example 5: Preparation of N'-methyl-3-(4-chlorobenzyloxy carbonylamino)benzamide (Compound No.27 in Table 1)

Melting point: 155-156°C
IR (KBr, cm⁻¹) : 3351, 3299, 1734, 1624, 1557.
NMR (DMSO - d₆, δ) : 2.74 (d, J = 4.5Hz, 3H), 5.14 (s, 2H), 7.25 - 7.43 (m, 6H), 7.55 (d, J = 8.3Hz, 1H), 7.91 (s, 1H), 8.32 (d, J = 4.5Hz, 1H), 9.88 (s, 1H)

### Example 6: Preparation of N'-methyl-3-(2,3-dichlorobenzyloxy carbonylamino)benzamide (Compound No.29 in Table 1)

Melting point: 167-168°C
IR (KBr, cm⁻¹) : 3401, 3258, 1744, 1711, 1649, 1561.
NMR (DMSO - d₆, δ) : 2.74 (d, J = 4.4Hz, 3H), 5.26 (s, 2H), 7.25 - 7.43 (m, 3H), 7.50 - 7.60 (m, 2H), 7.64 (d, J = 8.0Hz, 1H), 7.93 (s, 1H), 8.32 (d, J = 4.4Hz, 1H), 9.97 (s, 1H)

### Example 7: Preparation of N'-methyl-3-(2,6-dichlorobenzyloxy carbonylamino)benzamide (Compound No.35 in Table 1)

Melting point: 219-220°C
IR (KBr, cm⁻¹) : 3380, 3241, 1717, 1651, 1562.
NMR (DMSO - d₆, δ) : 2.74 (d, J = 4.3Hz, 3H), 5.35 (s, 2H), 7.25 - 7.60 (m, 6H), 7.92 (s, 1H), 8.35 (d, J = 4.3Hz, 1H), 9.92 (s, 1H)

### Example 8: Preparation of N'-methyl-3-(2-bromobenzyloxy carbonylamino)benzamide (Compound No.41 in Table 1)

Melting point: 163-164°C
IR (KBr, cm⁻¹) : 3324, 1728, 1622, 1559.
NMR (DMSO - d₆, δ) : 2.73 (d, J = 4.0Hz, 3H), 5.19 (s, 2H), 7.24 - 7.75 (m, 7H), 7.93 (s, 1H), 8.35 (d, J = 4.0Hz, 1H), 9.98 (s, 1H)

### Example 9: Preparation of N'-methyl-3-(2-methylbenzyloxy carbonylamino)benzamide (Compound No.47 in Table 1)

Melting point: 163°C
IR (KBr, cm⁻¹) : 3358, 3312, 1734, 1622, 1557.
NMR (DMSO - d₆, δ) : 2.35 (s, 3H), 2.76 (d, J = 4.4Hz, 3H), 5.17 (s, 2H), 7.18 - 7.35 (m, 3H), 7.35 - 7.45 (m, 3H), 7.57 (d, J = 7.7Hz, 1H), 7.94 (s, 1H), 8.37 (d, J = 4.4Hz, 1H), 9.89 (s, 1H)

### Example 10: Preparation of N'-methyl-3-(3-methylbenzyloxy carbonylamino)benzamide (Compound No.49 in Table 1)

Melting point: 155°C
IR (KBr, cm⁻¹) : 3343, 3279, 1736, 1624, 1559.
NMR (DMSO - d₆, δ) : 2.32 (s, 3H), 2.76 (d, J = 4.2Hz, 3H), 5.12 (s, 2H), 7.10 - 7.45 (m, 6H), 7.57 (d, J = 8.0Hz, 1H), 7.94 (s, 1H), 8.36 (d, J = 4.2Hz, 1H), 9.89 (s, 1H)

### Example 11: Preparation of N'-methyl-3-(4-isopropylbenzyloxy carbonylamino)benzamide (Compound No.57 in Table 1)

Melting point: 189-190°C
IR (KBr, cm⁻¹) : 3380, 3235, 1709, 1647, 1561.
NMR (DMSO - d₆, δ) : 1.19 (d, J= 6.8Hz, 6H), 2.76 (d, J= 3.9Hz, 3H), 2.88 (m, 1H), 5.12 (s, 2H), 7.20 - 7.40 (m, 6H), 7.57 (d, J = 7.7Hz, 1H), 7.93 (s, 1H), 8.35 (d, J = 3.9Hz, 1H), 9.87 (s, 1H)

### Example 12: Preparation of N'-methyl-3-(2-methoxybenzyloxy carbonylamino)benzamide (Compound No.61 in Table 1)

Melting point: 173°C
IR (KBr, cm⁻¹) : 3341, 3266, 1726, 1624, 1561.
NMR (DMSO - d₆, δ) : 2.76 (d, J = 4.0Hz, 3H), 3.82 (s, 3H), 5.14 (s, 2H), 6.99 (dd, J = 7.4Hz, 7.4Hz, 1H), 7.04 (d, J = 8.2Hz, 1H), 7.28 - 7.42 (m, 4H), 7.56 (d, J = 8.2Hz, 1H), 7.94 (s, 1H), 8.36 (d, J = 4.0Hz, 1H), 9.89 (s, 1H)

### Example 13: Preparation of N'-methyl-3-(4-methoxybenzyloxy carbonylamino)benzamide (Compound No.65 in Table 1)

Melting point: 158-159°C
IR (KBr, cm⁻¹) : 3331, 3295, 1730, 1613, 1555.
NMR (DMSO - d₆, δ) : 2.74 (d, J = 4.3Hz, 3H), 3.73 (s, 3H), 5.06 (s, 2H), 6.92 (d, J = 8.2Hz, 2H), 7.20 - 7.40 (m, 4H), 7.54 (d, J = 7.7Hz, 1H), 7.90 (s, 1H), 8.30 (d, J= 4.3Hz, 1H), 9.78 (s, 1H)

### Example 14: Preparation of N'-methyl-3-(4-chloro-2-nitro benzyloxycarbonylamino)benzamide (Compound.No.77 in Table 1)

Melting point: 193°C
IR (KBr, cm⁻¹) : 3366, 3248, 1717, 1624, 1562, 1537.
NMR (DMSO - d₆, δ) : 2.76 (d, J = 3.3Hz, 3H), 5.48 (s, 2H), 7.36 (dd, J = 7.8Hz, 7.8Hz, 1H), 7.44 (d, J = 7.8Hz, 1H), 7.58 (d, J = 7.8Hz, 1H), 7.77 (d, J = 8.1Hz, 1H), 7.90 - 7.98 (m, 2H), 8.23 (s, 1H), 8.35 (d, J = 3.3Hz, 1H), 10.04 (s, 1H)

### Example 15: Preparation of N'-methyl-3-(1-naphthylmethoxy carbonylamino)benzamide (Compound No.81 in Table 1)

Melting point: 228-229°C
IR (KBr, cm⁻¹) : 3353, 3285, 1730, 1626, 1555.
NMR (DMSO - d₆, δ) : 2.76 (d, J = 4.4Hz, 3H), 5.64 (s, 2H), 7.30 - 7.45 (m, 2H), 7.50 - 7.70 (m, 5H), 7.90 - 8.03 (m, 3H), 8.12 (d, J = 7.6Hz, 1H), 8.38 (d, J = 4.4Hz, 1H), 9.87 (s, 1H)

### Example 16: Preparation of N'-methyl-3-(2-naphthylmethoxy carbonylamino)benzamide (Compound No.89 in Table 1)

Melting point: 157-158°C
IR (KBr, cm⁻¹) : 3314, 1699, 1642, 1589, 1539.
NMR (DMSO - d₆, δ) : 2.76 (d, J = 4.8Hz, 3H), 5.32 (s, 2H), 7.23 - 7.42 (m, 2H), 7.42 - 7.60 (m, 4H), 7.82-7.98 (m, 5H), 8.32 (d, J = 4.8Hz, 1H), 9.90 (s, 1H)

### Example 17: Preparation of N'-methyl-3-(5-fluorenylmethoxy carbonylamino)benzamide (Compound No.99 in Table 1)

Melting point: 217°C
IR (KBr, cm⁻¹) : 3349, 3289, 1730, 1624, 1586, 1557.
NMR (DMSO - d₆, δ) : 2.76 (d, J = 4.2Hz, 3H), 4.32 (t, J = 6.6Hz, 1H), 4.48 (d, J = 6.6Hz, 2H), 7.25 - 7.50 (m, 6H), 7.59 (m, 1H), 7.76 (d, J = 7.2Hz, 2H), 7.92 (d, J = 7.2Hz, 2H), 7.93 (s, 1H), 8.37 (d, J = 4.2Hz, 1H), 9.87 (s, 1H)

### Example 18: Preparation of N'-methyl-3-(phenoxycarbonylamino) benzamide (Compound No.181 in Table 1)

Melting point: 193°C
IR (KBr, cm⁻¹) : 3401, 3268, 1753, 1624, 1555.
NMR (DMSO - d₆, δ) : 2.77 (d, J = 3.6Hz, 3H), 7.20 - 7.35 (m, 3H), 7.35 - 7.45 (m, 4H), 7.49 (d, J = 7.8Hz, 1H), 7.63 (s, 1H), 7.99 (s, 1H), 10.38 (s, 1H)

### Example 19: Preparation of

### 1-(3-methylcarbamoylphenyl)-3-phenylurea (Compound No.182 in Table 1)

After dissolving 209 mg of phenylisocyanate and 239 mg of 3-aminobenzoylmethylamide in 2 ml of dimethylformamide and stirring for 6 hours at room temperature, 15 ml of dilute hydrochloric acid was added. The obtained crystals were filtered and washed with water to obtain crude crystals. The crude crystals were dried under reduced pressure and added to 8 ml of ethyl acetate, and the mixture was heated under reflux for 10 minutes. The mixture was cooled to room temperature, and the crystals were collected by filtration and washed with ethyl acetate to obtain 386 mg of the target compound (90% yield) .

Melting point: 209-210°C
IR (KBr, cm⁻¹) : 3328, 3279, 1699, 1626, 1557.
NMR (DMSO - d₆, δ) : 2.75 (d, J = 4.1Hz, 3H), 6.95 (dd, J = 7.3Hz, 7.3Hz, 1H), 7.20 - 7.45 (m, 6H), 7.57 (d, J = 7.7Hz, 1H), 7.86 (s, 1H), 8.37 (d, J= 4.1Hz, 1H), 8.67 (s, 1H), 8.79 (s, 1H).

Compounds for Examples 20 and 21 were synthesized in the same manner as Example 19. The physical properties are listed below.

### Example 20: Preparation of

### 3-benzyl-1-(3-methylcarbamoylphenyl) urea (Compound No.5 in Table 1)

Melting point: 189-190°C
IR (KBr, cm⁻¹) : 3366, 3333, 1640, 1559.
NMR (DMSO-d₆, δ) : 2.73 (d, J = 4.4Hz, 3H), 4.28 (d, J= 5.9Hz, 2H), 6.62 (t, J = 5.9Hz, 1H), 7.15 - 7.40 (m, 7H), 7.54 (d, J = 7.3Hz, 1H), 7.79 (s, 1H), 8.28 (d, J = 4.4Hz, 1H), 8.66 (s, 1H).

### Example 21: Preparation of 3-benzyl-1-(3-methylcarbamoylphenyl) thiourea (Compound No.6 in Table 1)

Melting point: 199°C
IR (KBr, cm⁻¹) : 3343, 3246, 3069, 1630, 1584, 1528.
NMR (DMSO - d₆, δ) : 2.76 (d, J = 4.5Hz, 3H), 4.72 (t, J = 5.4Hz, 2H), 7.20 - 7.40 (m, 6H), 7.45 - 7.60 (m, 2H), 7.81 (s, 1H), 8.20 (s, 1H), 8.36 (d, J = 4.5Hz, 1H), 9.65 (s, 1H).

### Example 22: Preparation of N'-methyl-3-(2-(2-methyl-5-nitro-1-imidazolyl))ethoxycarbo nylamino)benzamide (Compound No.233 in Table 1)

Melting point: 207°C
IR (KBr, cm⁻¹) : 3362, 1734, 1636, 1591, 1533.
NMR (DMSO - d₆, δ) : 2.48 (s, 3H), 2.76 (d, J = 4.2Hz, 3H), 4.47 (t, J = 4.8Hz, 2H), 4.61 (t, J = 4.8Hz, 2H), 7.34 (dd, J = 7.7Hz, 7.5Hz, 1H), 7.43 (d, J = 7.5Hz, 1H), 7.55 (d, J = 7.7Hz, 1H), 7.85 (s, 1H), 8.05 (s, 1H), 8.34 (d, J = 4.4Hz, 1H), 9.76 is, 1H).

### Test Example 1: Measurement of anti-Helicobacter pylori activity

A 5 ml portion of brain heart infusion culture medium containing 10% fetal bovine serum (Difco) was dispensed in a test tube, and then the medium was inoculated with human-isolated *Helicobacter pylori* strain 31A (obtained from the Tokyo Metropolitan Health Institute, Microorganism Department, First Bacteria Laboratory) , and shake culturing was carried out under slightly aerobic conditions (5% oxygen, 10% carbon dioxide, 85% nitrogen) at 37°C for 48 hours.

The culture solution was then inoculated into brain heart infusion medium containing 10% fetal bovine serum at a ratio of 5%, and after adding a test compound dissolved in 10% dimethyl sulfoxide and shake culturing under slightly aerobic conditions at 37°C for 48 hours, the growth of *Helicobacter pylori* was examined. The antibacterial activity was recorded as the lowest concentration that exhibited growth inhibition (minimum inhibitory concentration: MIC). The results are shown in Table 2.

**Table 2**

| Anti-*Helicobacter pylori* activity | |
|---|---|
| Example No. Compound (Compound No. in Table 1) | MIC (µg/ml) |
| 1 (No. 51) | 0.10 |
| 2 (No. 17) | 0.78 |
| 3 (No. 21) | 0.78 |
| 4 (No. 23) | 0.39 |
| 5 (No. 27) | 0.20 |
| 6 (No. 29) | 0.20 |
| 7 (No. 35) | 0.20 |
| 8 (No. 41) | 0.39 |
| 9 (No. 47) | 0.39 |
| 10 (No. 49) | 0.39 |
| 11 (No. 57) | 0.78 |
| 12 (No. 61) | 0.78 |
| 13 (No. 65) | 0.78 |
| 15 (No. 81) | 0.05 |
| 16 (No. 89) | 0.10 |
| 20 (No. 5) | 1.56 |

The results in Table 2 demonstrate that the compounds of the present invention have potent inhibitory activity against *Helicobacter pylori.*

### Test Example 2: Formulation Examples

### (1) Tablet

The following ingredients were mixed according to a conventional method, and compressed to obtain a tablet using a conventional apparatus

| | |
|---|---|
| Compound of Example 1 | 100 mg |
| Crystalline cellulose | 180 mg |
| Corn starch | 300 mg |
| Lactose | 600 mg |
| Magnesium stearate | 15 mg |

### (2) Soft capsule

The following ingredients were mixed according to a conventional method and filled into a soft capsule.

| | |
|---|---|
| Compound of Example 1 | 100 mg |
| Olive oil | 900 mg |
| Lecithin | 60 mg |

### Industrial Applicability

The compounds of the present invention exhibit potent antibacterial activity against *Helicobacter pylori,* and are therefore useful for the prevention or treatment of gastritis, gastric ulcer, duodenal ulcer, gastric cancer, duodenal cancer and malignant gastric lymphoma, and as agents to prevent recurrence of gastritis, gastric ulcer and duodenal ulcer.

## Claims

1. An amide derivative represented by the following general formula (I) or its pharmaceutically acceptable salt, or a solvate thereof or a hydrate thereof. 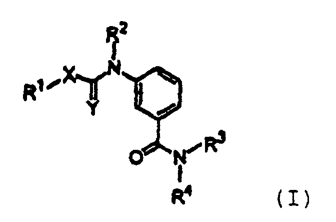 (wherein R¹ represents [1] a C₁-C₁₀ alkyl group optionally substituted with an optionally substituted C₆-C₁₄ aryl group, fluorenyl group or heterocycle residue, [2] an optionally substituted C₆-C₁₄ aryl group or [3] an optionally substituted heterocycle residue; R², R³ and R⁴ each independently represent a hydrogen atom or a C₁-C₅ alkyl group; X represents an oxygen atom or N-R⁵ (where R⁵ represents a hydrogen atom or a C₁-C₅ alkyl group); and Y represents an oxygen atom or sulfur atom (excluding compounds wherein R¹ is benzyl group, R² and R³ are a hydrogen atom, R⁴ is propyl group and X and Y are an oxygen atom.)

2. A compound according to Claim 1, **characterized in that** R² and R³ represent a hydrogen atom, and X represents an oxygen atom or N-H.

3. A compound according to Claim 1 or 2, **characterized in that** R⁴ represents a methyl group.

4. A compound according to Claim 3, **characterized in that** Y represents an oxygen atom.

5. A compound according to Claim 4, **characterized in that** R¹ represents a C₁-C₁₀ alkyl group optionally substituted with an optionally substituted C₆-C₁₄ aryl group or an optionally substituted heterocycle residue.

6. A compound according to Claim 4, **characterized in that** R¹ represents a C₁-C₅ alkyl group optionally substituted with an optionally substituted C₆-C₁₄ aryl group or an optionally substituted heterocycle residue.

7. A compound according to Claim 4, **characterized in that** R¹ represents a methyl group optionally substituted with an optionally substituted C₆-C₁₄ aryl group or an optionally substituted heterocycle residue.

8. A compound according to Claim 4, **characterized in that** R¹ represents a methyl group optionally substituted with an optionally substituted C₆-C₁₄ aryl group.

9. A compound according to Claim 4, **characterized in that** R¹ represents a methyl group optionally substituted with an optionally substituted heterocycle residue.

10. A compound selected from the group consisting of N'-methyl-3-(2-chlorobenzyloxycarbonylamino)benzamide, N'-methyl-3-(4-chlorobenzyloxycarbonylamino)benzamide, N'-methyl-3- (2, 3-dichlorobenzyloxycarbonylamino) benzamide, N'-methyl-3-(2,6-dichlorobenzyloxycarbonylamino)benzamide, N'-methyl-3-(2-bromobenzyloxycarbonylamino)benzamide, N'-methyl-3-(2-methylbenzyloxycarbonylamino)benzamide, N'-methyl-3-(3-methylbenzyloxycarbonylamino)benzamide, N'-methyl-3-(4-methylbenzyloxycarbonylamino)benzamide, N'-methyl-3-(1-naphthylmethoxycarbonylamino)benzamide, and N'-methyl-3-(2-naphthylmethoxycarbonylamino)benzamide or its pharmaceutically acceptable salt, or a solvate thereof or a hydrate thereof.

11. A medicament comprising as an active ingredient a compound selected from the group consisting of a compound according to any one of claims 1 to 10.

12. The medicament according to Claim 11 which has antibacterial activity.

13. The medicament according to Claim 12 which has antibacterial activity against bacteria belonging to the genus *Helicobacter.*

14. The medicament according to Claim 13 which has anti-*Helicobacter pylori* activity.

15. The medicament according to Claim 11 for the prevention and/or treatment of an digestive disease selected from the group consisting of gastritis, gastric ulcer, duodenal ulcer, gastric cancer, duodenal cancer, malignant lymphoma and MALT lymphoma.

16. The medicament according to Claim 11 for prevention of recurrence of gastritis, gastric ulcer or duodenal ulcer.
